**Europäisches Patentamt**

**European Patent Office**

**Office européen des brevets**

(19)

(11) Publication number: **0 076 422**
A2

(12) # EUROPEAN PATENT APPLICATION

(21) Application number: **82108673.3**

(22) Date of filing: **20.09.82**

(51) Int. Cl.³: **A 61 M 1/03**
**B 01 D 13/00**

(30) Priority: **05.10.81 SE 8105851**

(43) Date of publication of application:
**13.04.83 Bulletin 83/15**

(84) Designated Contracting States:
**AT BE CH DE FR GB IT LI LU NL**

(71) Applicant: **GAMBRO AB**
**Box 10101**
**S-220 10 Lund(SE)**

(72) Inventor: **Christopherson, Kjell**
**Tamburinvägen 10**
**S-245 00 Staffanstorp(SE)**

(72) Inventor: **Falkvall, Thore**
**Karl X Gustafs gata 67**
**S-252 40 Helsingborg(SE)**

(72) Inventor: **Mattisson, Ulf**
**Ollonborrevägen 11**
**S-240 17 S. Sandby(SE)**

(74) Representative: **Boberg, Nils Gunnar Erik et al,**
**HOLGER CRAFOORD AB Patent Department Box 10101**
**S-220 10 Lund(SE)**

(54) Filtering device.

(57) A filtering device comprising an inlet (8) and an outlet (9) for the liquid filtered and an outlet (11) for the filtrate and with the filtration being arranged so that it takes place in two chambers (2,3) connected in series, which consist at least partly of membrane material through which the filtration is taking place.

The device is intended primarily to be used in connection with medical treatment of blood and here in particular in connection with haemofiltration or plasmafers, in which methods a thickening of the blood and consequently a pressure drop occurs.

The characteristic feature of the invention is an inlet (19) for replacement liquid, which is adapted so as to introduce this liquid between the said two chambers (2,3).

./...

Fig.1

TITLE

FILTERING DEVICE

TECHNICAL FIELD

The present invention relates to a filtering device comprising an inlet and an outlet for the liquid filtered and an outlet for .the filtrate, the filtration being arranged so as to take place in two chambers connected in series, which consist at least partly of membrane material through which the filtration is taking place.

The invention is intended primarily to be used in connection with medical treatment of blood and here in particular with haemofiltration or plasmafers, in which methods.a thickening of the blood and consequently a pressure drop occurs between the inlet and the outlet of the liquid filtered.

Haemofiltration thus refers to a process wherein the blood fluid is withdrawn from the patient together with toxins which could not be removed by means of the normal renal function. Since a quantity of liquid in the order of magnitude of 20 litres is withdrawn from the patient in each case of treatment, substantially the same quantity of replacement liquid is supplied, insofar as a certain reduction in weight is not desired..-

Plasmafers refer to a process wherein only the blood plasma is withdrawn from the blood donor and the blood cells and similar larger molecules, together with a small portion of the blood plasma, are returned. In this process too a certain quantity of replacement liquid may be supplied to the patient.

Both, haemofiltration and plasmafers, take place appropriately with the help of membrane filtration in devices which resemble normal membrane dialysers. Usually, however, more permeable membranes and higher pressures are used. Moreover, of course, no dialysis is to be found on the side of the membrane remote from the blood.

BACKGROUND ART

Normally separate filtering devices are used for processes of the abovementioned type which are thus similar to normal membrane dialysers. Tests have also been carried out with filtering devices coupled in parallel or in series. Especially suitable is a connection in series when an increase in the pressure drop is obtained which
in turn gives an increase in the amount of filtrate. However, if
the pressure drop across the filtering device is too great, problems
may arise. For example, the blood may be damaged through haemolysis.

Experiments have also been carried out with chambers connected
in series and in parallel arranged in one and the same casing. See
for example US patent specification 4 038 190. Difficulties may also
be experienced, however, with the designs described in this patent
if, in particular for haemofiltration or plasmafers, a sufficiently
high pressure is to be achieved without the risk of too high a presure
when, for example, the filter becomes partially choked.


DISCLOSURE OF INVENTION

The abovementioned problem is solved in accordance with the
invention with the help of a filtering device comprising an inlet
and an outlet for the liquid filtered and an outlet for the filtrate,
the filtration being arranged to take place in two chambers connected
in series, which consist at least partly of membrane material through
which the filtration is taking place.

The characteristic feature of the invention is an inlet for
replacement liquid, which is adapted so as to introduce the replacement liquid between the said two chambers.

It is appropriate to arrange a further inlet for replacement
liquid to introduce the same after the filtration into the second chamber. This makes it possible to supply before the second chamber only
such a quantity of replacement liquid that the conditions of flow in this
chamber remain ideal.

It is also possible to arrange a further inlet for replacement
liquid to introduce the same before the filtration into the first
chamber. This makes it possible to influence the conditions of flow
also in this chamber.

The filtering device in accordance with the invention may be
of the so-called plane film type or it may consist of a membrane
tube wound into a spiral. In a preferred embodiment, however, it
is of the fibrous type, both chambers being arranged in one and the
same casing and each being formed of a number of interior spaces of
thin-walled fibres. In filtering devices of this type a relatively high pressure can be used whilst the risk of a leakage through membrane ruptures remains small.

The two chambers are preferably arranged in one and the same casing. In this way a compact design can be achieved which with the help of a simple system of flexible tubes can be connected to the patient on the one hand and to a control apparatus, comprising pumps, pressure monitors and other supervisory devices, on the other hand.

The fibres of the two chambers may be cast, for example, into two end walls of a cylindrical casing which, outside these end walls, has outer covers with the said inlet and outlet for the liquid treated.

The said inlet and outlet are arranged preferably in one of the outer covers, separated by a seal arranged between the adjoining end wall and the cover, whilst the other outer cover comprises an inlet for the replacement liquid.

If the filtering device in accordance with the invention comprises at least two inlets for replacement liquid, these inlets should be connected or be connectable to a common supply line via a distributing valve. This distributing valve may be adapted so that it conducts quantities determined beforehand to the respective inlet, substantially regardless of the pressure prevailing at these inlets. In this manner ideal conditions of flow can be produced, especially in the second chamber, where otherwise as a rule difficulties arise.

A simple design, applicable in practice, is obtained if the distributing valve is adapted so as to conduct substantially equal quantities to the respective inlets.

For a further controlling of the conditions in the different chambers, preferably controllable valves can be arranged between the distributing valve and one or more of the inlets. It is preferable to arrange such a controllable valve between each one of the inlets and the distributing valve.

The distributing valve preferably comprises a coupling onto which a supply line is slipped in such a manner that it covers the openings to two internal ducts which are connected to the respective inlets for replacement liquid, the sectional area of flow between the supply line and the respective duct increasing at a higher back pressure in the latter.

BRIEF DESCRIPTION OF DRAWINGS

The invention will be described in more detail in the
following with reference to the enclosed drawings, which by
way of example show a preferred embodiment of the same.

Fig.1 shows a relatively complete construction in accordance with
the invention.

Figures 2 and 3 show a section and an end elevation respectively.
of a detail from figure 1 on an enlarged scale.

BEST MODE OF CARRYING OUT THE INVENTION

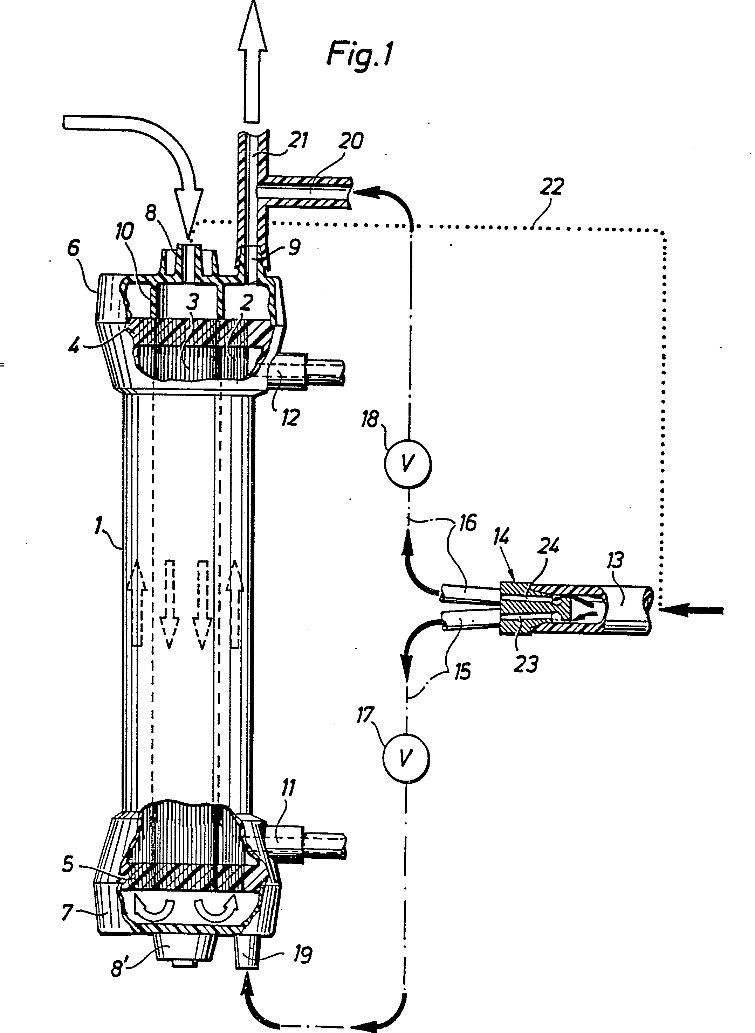

The design shown in figure 1 by way of an example comprises
a casing 1 with concentric fibre bundles 2 and 3 cast into it.
These fibre bundles are cast into end walls 4 and 5. Outside the
end walls outer covers 6 and 7 are fitted onto the casing. The
outer cover 6 is provided with an inlet for the liquid treated,
which in particular is constituted of blood. In the same manner,
the outer cover 7 is provided with an inlet 8'. In normal use,
however, the latter is plugged up and has been added primarily
from a point of view of manufacture. Through this extra inlet 8',
the two covers 6 and 7 can have the same shape and at the same time
the possibility of an effective scavenging of the construction in
connection with manufacture is provided.

An outlet for the filtrate is designated 9. The inlet 8 and
outlet 9 are separated in the example shown by a flange 10 adapted
so as to yield at a higher pressure. As a result the blood supplied
will flow from the inlet 8 through the internal central fibre bundle
3 and via the outer cover 7 back through the outer fibre bundle 2
to the outlet 9.

The filtrate is removed via an outlet 11, which too has a counterpart 12, plugged up during application, to allow an effective scavenging of the construction in connection with manufacture.

The replacement liquid is supplied in the construction shown
through a supply line 13 which is. slipped onto a coupling 14. From
this coupling the replacement liquid is conducted via lines 15 and
16 and valves 17 and 18 to a first inlet 19 and a second inlet 20
respectively for replacement liquid. The inlet 19, thus arranged
in the outer cover 7, supplies replacement liquid to the space between the two chambers 2 and 3 formed by the respective fibre bundles. The inlet 20 supplies replacement liquid instead to an outlet line 21 slipped onto the outlet 9.

A thir. possibility would be to supply replacement liquid to the inlet 8 for the liquid treated. This has been indicated schematically in fig.1 by the dotted line 22.

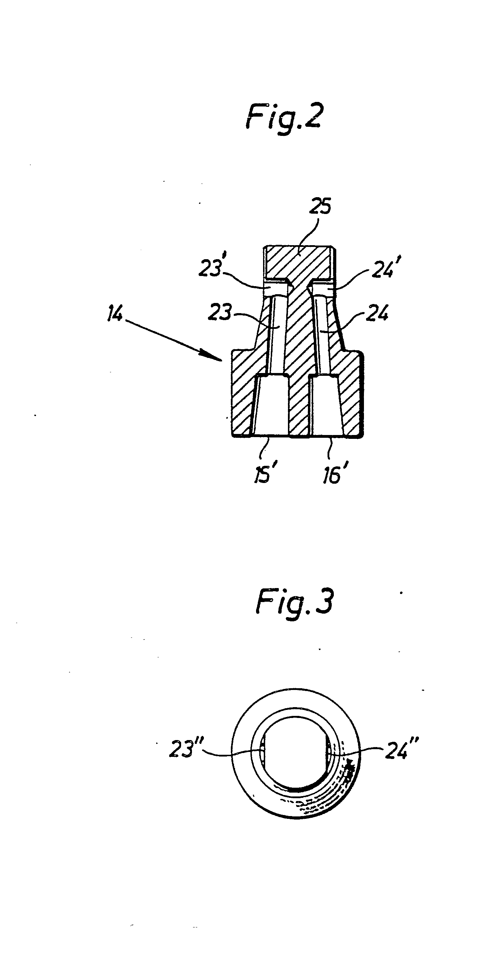

The more detailed design of the coupling 14 is evident from figures 2 and 3 where it can be seen on a larger scale. As can be seen, it comprises two ducts 23 and 24, whose openings 15' and 16' are intended to be connected to lines 15 and 16. The opposite openings 23' and 24' are intended to be covered at the same time by the supply line 13. This supply line 13 is thus intended to be slipped over the end spigot 25 of the coupling 14, so that it covers the openings 23' and 24'. At the place of these openings, the end spigot 25 is provided with two bevellings 23" and 24". Normally a certain flow will take place through these bevellings into the respective ducts. If an increased pressure arises in anyone of these ducts, the supply line 13 will expand so that the sectional area of flow to this duct increases. At the same time the pressure of the supply tube against the opening of the opposite duct increases, so that a tendency towards a reduced flow through the latter is obtained. If the pressure at the inlets 19 and 20 normally were to be equal, the design shown would substantially maintain the same flow to these two inlets also when there are variations in pressure. However, the pressure at the inlet 19 is normally higher, and if it is desired to obtain the same quantity of replacement liquid at the two inlets, a control has to be applied. This can be done either with the help of valves 17 and 18 or else the coupling 14 may simply be substituted by a similar one with different dimensions of the ducts 23 and 24. In this manner the conditions of flow in the ducts 15 and 16 can be varied within wide limits so as to comply to any demands for good operating conditions in the filtering device itself.

INDUSTRIAL APPLICABILITY

It is evident from what has been said in the foregoing that the invention is intended primarily to be used for the manufacture of membrane filtering devices for haemofiltration and plasmafers and similar medical

0076422

applications. However, it will·be clear to  those versed in the
art that the invention can also be applied in other cases where
a filtration is desired with simultaneous supply of replacement
liquid and in particular at a relatively high pressure.

Naturally, the invention is not limited exclusively to the
embodiment described above, but may be varied within the scope of
the following claims. See for example the patent application
81.05850-5 ...........submitted at the same time, which  is aimed in particular
at different designs of the seal 10 in order to perfect its function
as an overflow valve.

CLAIMS

1. A filtering device comprising an inlet (8) and an outlet (9) for the liquid filtered and an outlet (11) for the filtrate, the filtration being arranged    take place in two chambers (2,3) connected in series, which consist at least partly of membrane material through which the filtration is taking place, c h a - r a c t e r i z e d  by an inlet (19) for replacement liquid which is adapted so as to introduce the replacement liquid between the said two chambers (2,3).

2. A filtering device in accordance with claim 1, c h a r a c t e r - i z e d  by a further inlet (20) for replacement liquid which is adapted so as to introduce the replacement liquid after the filtration into the second chamber (2).

3. A filtering device in accordance with claim 1 or 2,c h a r a c t e r - i z e d  by a further inlet (22,8) for replacement liquid which is adapted so as to introduce replacement liquid before the filtration into the first chamber (3).

4. A filtering device in accordance with anyone of the preceding claims,c h a r a c t e r i z e d  in that the two chambers (2,3) are arranged in one and the same casing (1) and that each is formed of a number of interior spaces of thin-walled fibres.

5. A filtering device in accordance with claim 4, the fibres of both chambers (2,3) being cast into two end walls (4,5) of a cylindrical casing (1) which, outside these end walls, has outer covers (6,7) with the said inlet (8) and outlet (9) for the liquid treated, c h a r a c t e r i z e d in that the said inlet (8) and outlet (9) are arranged in one (6) of the outer covers (6,7),separated by a seal (10) arranged between the adjoining end wall (4) and the cover (6) whilst the other outer cover (7) comprises an inlet (19) for replacement liquid.

6. A filtering device in accordance with anyone of the preceding claims, comprising at least two inlets (19,20) for replacement liquid, c h a r a c t e r i z e d  in that these inlets (19,20) are connected or are connectable to a common supply line (13) via a distributing valve (13,14).

7. A filtering device in accordance with claim 6,c h a r a c t e r i z e d in that the distributing valve (13,14) is adapted so as to conduct quantities determined beforehand to the respective inlet (19,20),

-8-

0076422

substantially regardless of the pressure prevailing at these inlets.

8. A filtering device in accordance with claim 7,c h a r a c t e r - i z e d in that the distributing valve (13,14) is adapted so as to conduct substantially equal quantities to the respective inlets (19,20).

9. A filtering device in accordance with anyone of claims 6-8, c h a r a c t e r i z e d by a preferably controllable valve (17 or 18) arranged between the distributing valve (13,14) and one of the inlets (19 or 20).

10. A filtering device in accordance with claim 9,c h a r a c t e r - i z e d in that a controllable valve (17 or 18) is arranged between each of the inlets (19,20) and the distributing valve (13,14).

11. A filtering device in accordance with claim 6,c h a r a c t e r - i z e d in that the distributing valve (13,14) comprises a coupling (14) onto which is slipped a supply line (13) in such a manner that it covers the openings (23',24') to two internal ducts (23,24) which are connected to the respective inlet (19,20) for replacement liquid, the sectional area of flow between the supply line (13) and the respective duct (23,24) increasing at a higher back pressure in the latter.

12. A filtering device in accordance with claim 5,c h a r a c t e r - i z e d in that the two outer covers (6,7) are of identical design, the blood inlet (8') of one of the covers (7) being blocked up during usage.

0076422

Fig.1

Fig.2

Fig.3